# EUROPEAN PATENT APPLICATION

(11) **EP 4 625 305 A1**
(43) Date of publication of application: **01.10.2025**
(21) Application number: 23895054.7
(22) Date of filing: 23.11.2023
(51) Int. Cl.: G06Q 50/26, G06Q 50/10, G06Q 30/02, C12M 3/00, H04L 9/00

(54) **ECO-FRIENDLY BUSINESS SYSTEM AND BUSINESS METHOD FOR CARBON REDUCTION USING MICROALGAE**

(30) Priority: 23.11.2022 KR 20220158349
(71) Applicant: FORNATURES CO., LTD., Cheongju-si, Chungcheongbuk-do 28611 (KR)
(72) Inventor: RYU, Ho Lim, Chungcheongbuk-do 27825 (KR)
(74) Representative: Impuls legal PartG mbB
(86) International application number: PCT/KR2023/019026
(87) International publication number: WO 2024/112127

(57) **Abstract**

The disclosed carbon reduction eco-friendly business system utilizing microalgae comprises: a microalgae cultivation device that is equipped for each of a plurality of consumers to capture carbon and cultivate microalgae; a consumer terminal that is interconnected with the microalgae cultivation device by establishing a wireless communication network and calculates carbon reduction information based on at least one of a change in carbon concentration, a change in carbon dioxide concentration, and a harvest amount of microalgae received from the microalgae cultivation device; and a business server that provides a reward to the consumer terminal based on the carbon reduction information received through the consumer terminal and stores the carbon reduction information in a blockchain; wherein, when a business operating the business server harvests microalgae from the microalgae cultivation devices of the plurality of consumers and produces an eco-friendly microalgae product using the same, the plurality of consumers are equipped to purchase the microalgae product using the reward provided to the consumer terminal to establish a virtuous cycle business structure.

## Description

### Technical Field

The present disclosure relates to a carbon reduction eco-friendly business system and method utilizing microalgae. Specifically, it pertains to a system and method where consumers cultivate microalgae to reduce carbon emissions from their living spaces and receive rewards based on the amount of carbon reduced. The business operator collects the microalgae harvested by consumers, produces microalgae-based products, and provides these products back to the consumers, forming an eco-friendly circular structure.

### Background Art

This section provides background information related to the present disclosure, which is not necessarily prior art.

Currently, global warming is accelerating, and the 2030s are expected to see an average temperature rise of 2-3°C. Carbon dioxide (CO₂) is the greenhouse gas that has the most significant impact on global warming.

The atmospheric concentration of CO₂ was approximately 180 ppm during the last ice age. However, since the Industrial Revolution, it has risen sharply, reaching 290 ppm by 1890 and about 360 ppm by 1992, marking a 30% increase over a century. By the end of the 21st century, it is expected to reach 500 ppm, twice the pre-industrial level.

The pressing issue of greenhouse gas reduction, highlighted as a top environmental concern by the international community, underscores the critical need for proactive measures, programs, and the development and dissemination of CO₂ control technologies.

Among the technologies for CO₂ fixation, biological methods using photosynthetic microorganisms have been proposed in various forms. Specifically, the use of microalgae for CO₂ treatment and the production of valuable byproducts from biomass generated in the process have been actively researched to create added value.

In this context, prior technologies have included systems aimed at significantly enhancing microalgae cultivation efficiency and providing cultivation devices with excellent water circulation for microalgae. An example of such prior art is Korean Patent Publication No. 10-2016-0136623, titled "Microalgae Cultivation Device."

Microalgae are among the oldest organisms on Earth, with thousands of species identified, although only about 0.1% of their bioactivity is known. Consequently, only a small fraction is cultivated on an industrial scale. Notable examples include Chlorella and Spirulina, which are used in various applications such as dietary supplements, health foods, aquaculture feed, alternative medicines, and energy resources.

Microalgae inhabit freshwater or seawater and are unicellular plants without roots, stems, or leaves, performing photosynthesis with chlorophyll. They contain plant-based fatty acids, proteins, minerals, and various vitamins, making them highly valuable for health food products. Additionally, their rapid growth and reproduction under suitable environmental conditions allow for large-scale harvesting within a short time, offering immense potential as a biodiesel feedstock.

However, existing microalgae cultivation devices and systems primarily focus on improving the efficiency of microalgae cultivation, without providing specific details on how microalgae can be utilized. As a result, general users find it challenging to recognize the direct connection between microalgae cultivation and environmental benefits, making it difficult for them to perceive the necessity of such cultivation.

Accordingly, there is a need for methods that enable the general public to take an interest in the environment and actively participate in reducing carbon emissions, and methods that utilize microalgae cultured to capture carbon dioxide are required.

### Description of the invention

### Technical Problem

The present disclosure aims to provide a carbon reduction eco-friendly business system and method utilizing microalgae, which establish an eco-friendly circular business structure. Through microalgae cultivation, consumers purify air and reduce CO₂ in their living spaces, receiving rewards in return. Business operators harvest the cultivated microalgae to produce and sell eco-friendly products, while consumers use the received rewards to purchase these products, thereby forming a sustainable circular system.

### Solution to Problem

This section provides a general summary of the disclosure and is not a comprehensive disclosure of its full scope or all of its features.

In order to solve the above problem, a carbon reduction eco-friendly business system utilizing microalgae according to one of the aspects described in the present invention comprises: a microalgae cultivation device that is equipped for each of a plurality of consumers to capture carbon and cultivate microalgae; a consumer terminal that is interconnected with the microalgae cultivation device by establishing a wireless communication network and calculates carbon reduction information based on at least one of a change in carbon concentration, a change in carbon dioxide concentration, and a harvest amount of microalgae received from the microalgae cultivation device; and a business server that provides a reward to the consumer terminal based on the carbon reduction information received through the consumer terminal and stores the carbon reduction information in a blockchain; wherein, when a business operating the business server harvests microalgae from the microalgae cultivation devices of the plurality of consumers and produces an eco-friendly microalgae product using the same, the plurality of consumers are equipped to purchase the microalgae product using the reward provided to the consumer terminal to establish a virtuous cycle business structure.

In the carbon reduction eco-friendly business system utilizing microalgae according to one aspect of the present invention, the change in carbon concentration is detected by detecting a change between the concentration of carbon contained in air supplied to the microalgae culture device and the concentration of carbon contained in air discharged through the microalgae culture device.

In the carbon reduction eco-friendly business system utilizing microalgae according to one aspect of the present invention, the change in carbon dioxide concentration is detected by detecting a change in carbon dioxide concentration in a living space where the microalgae cultivation device is installed while the microalgae cultivation device performs microalgae cultivation.

In the carbon reduction eco-friendly business system utilizing microalgae according to one aspect of the present invention, the microalgae cultivation device accommodates a culture medium prepared for culturing microalgae in the form of an aqueous liquid or solid inside, inoculates a specific microalgae spawn into the accommodated culture medium, and performs cultivation and harvesting of the specific microalgae spawn by providing air and light, thereby generating information on at least one of a change in carbon concentration, a change in carbon dioxide concentration, and a microalgae yield.

In the carbon reduction eco-friendly business system utilizing microalgae according to one aspect of the present invention, the consumer terminal updates the status of the microalgae cultivation device in real time, monitors the microalgae cultivation amount and carbon capture data, and controls the mode.

In the carbon reduction eco-friendly business system utilizing microalgae according to one aspect of the present invention, the consumer terminal monitors the microalgae cultivation device according to cultivation standard information on the change pattern of appropriate cultivation conditions according to the cultivation status and cultivation time of each type of microalgae and the harvest time, and shares information on the microalgae cultivation device according to the cultivation standard information with the business server.

A carbon reduction eco-friendly business method utilizing microalgae according to another aspect of the present invention, comprises the steps of: capturing carbon through a microalgae cultivation device at a location designated by a consumer and cultivating microalgae; calculating carbon reduction information based on at least one of a change in carbon concentration, a change in carbon dioxide concentration, and a microalgae harvest amount due to the cultivation of the microalgae; storing the carbon reduction information in a blockchain by a business server; and providing a reward according to the carbon reduction amount to the consumer terminal; a step where the business operator operating the business server harvests the microalgae cultured in the microalgae cultivation device, produces eco-friendly microalgae products using the harvested microalgae, and sells them online and offline; and a step where the consumer purchases the microalgae product with the reward provided to the consumer terminal.

In the carbon reduction eco-friendly business method utilizing microalgae according to another aspect of the present invention, the reward provided to the consumer terminal is in the form of points.

In the carbon reduction eco-friendly business method utilizing microalgae according to another aspect of the present invention, the microalgae cultivation device is managed by a business server and provided in a rental form to enable the business to harvest microalgae.

### Advantageous Effect of the Invention

According to the present invention, consumers purify the air in their living spaces through microalgae cultivation using a microalgae cultivation device provided by business operators, and receive rewards based on the amount of carbon reduced in the process. Business operators can harvest microalgae cultivated by consumers without a separate microalgae cultivation space and produce and sell eco-friendly products utilizing microalgae. Consumers can use the rewards received to purchase eco-friendly products, thereby providing an eco-friendly virtuous cycle business structure with a virtuous cycle structure.

In addition, according to the present invention, by storing microalgae culture data and reduced carbon amount in a blockchain, there is an effect of preventing falsification of data and transparently proving carbon reduction data.

### Brief description of the drawing

Figure 1 is a block diagram showing a carbon reduction eco-friendly business system utilizing microalgae according to an embodiment of the present invention.
Figure 2 is an example of a screen displayed on a consumer terminal of a carbon reduction eco-friendly business system utilizing microalgae according to an embodiment of the present invention.
Figure 3 is a flow chart showing a carbon reduction eco-friendly business method utilizing microalgae according to an embodiment of the present invention.
FIG. 4 is a diagram showing a virtuous cycle structure between consumers and business operators in a carbon reduction eco-friendly business method utilizing microalgae according to an embodiment of the present invention.

### Detailed description of the invention

Hereinafter, an embodiment of a carbon reduction eco-friendly business system utilizing microalgae according to the present invention will be described in detail with reference to the drawings.

However, the intrinsic technical idea of the present invention cannot be said to be limited in its possible implementation by the embodiments described below, and it is disclosed that it encompasses the scope that can be easily proposed by a person skilled in the art by replacing or changing the embodiments described below based on the intrinsic technical idea of the present invention.

In addition, the terms used below have been selected for convenience of explanation, and therefore, in understanding the intrinsic technical idea of the present invention, they should be appropriately interpreted in a meaning that is consistent with the technical idea of the present invention, without being limited to the dictionary meaning.

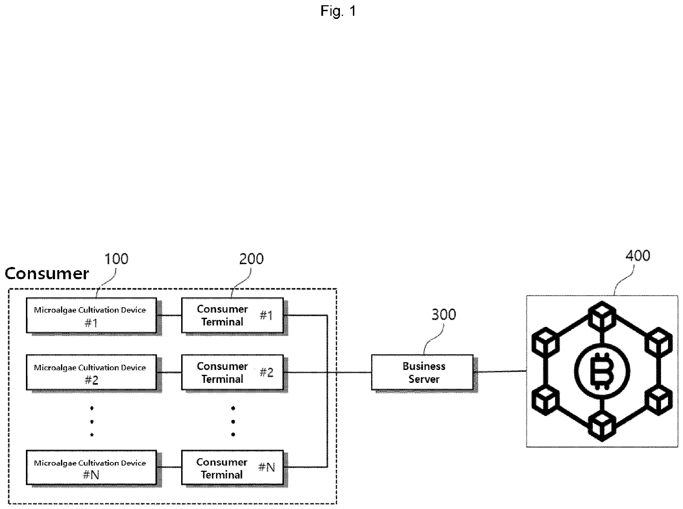

Figure 1 is a block diagram showing a carbon reduction eco-friendly business system utilizing microalgae according to an embodiment of the present invention.

As shown in Figure 1, the carbon reduction eco-friendly business system utilizing microalgae consists of a microalgae cultivation device (100), a consumer terminal (200), a business server (300), and a blockchain (400).

Multiple consumers place microalgae cultivation devices (100) in their respective living spaces.

The air in the living space is purified as it passes through the microalgae cultivation device (100).

As the air in the living space passes through the microalgae cultivation device 100, microalgae are cultivated in the microalgae cultivation device (100).

As a result, the carbon contained in the air in the living space is captured, that is, the amount of carbon contained in the air in the living space is reduced.

The microalgae cultivation device (100) is equipped with a microalgae culture medium inoculated with the desired microalgae spawn.

The microalgae culture medium is accommodated in a suitable storage space in a liquid or solid state.

Additionally, it is equipped to provide air and light to the microalgae culture medium.

By this, it is equipped to cultivate microalgae and harvest the cultivated microalgae.

At this time, the system according to the present invention is required to calculate the amount of carbon captured during the cultivation process of microalgae, which is the basis for the reward provided to the consumer terminal.

The amount of carbon captured is calculated by detecting and comparing the amount of carbon contained in the air supplied to the microalgae culture medium and the amount of carbon contained in the air discharged through the microalgae culture medium.

To this end, sensors that detect the amount of carbon are provided at the input side through which air supplied to the microalgae culture medium passes, and at the output side through which air discharged through the microalgae culture medium passes.

A sensor for detecting the amount of carbon dioxide is provided to detect the amount of carbon dioxide contained per unit volume of air.

In contrast, a sensor that detects the carbon dioxide concentration is installed in the living space where the microalgae cultivation device (100) is located, and while the microalgae cultivation device (100) is operating, the carbon dioxide concentration in the living space is measured at set time intervals to obtain the amount of change in the carbon dioxide concentration, and the amount of captured carbon can be derived therefrom.

In another way, carbon reduction information is calculated based on the yield of microalgae harvested from the microalgae cultivation device (100). At this time, the carbon reduction amount can be calculated using calculation formula information based on the carbon capture amount compared to the microalgae yield.

To this end, the microalgae cultivation device (100) may further include a detection unit that detects the harvest amount of microalgae.

In the system according to the present invention, the microalgae cultivation device (100) may comprise a cultivation body, an air supply module, a light irradiation module, and a microalgae harvesting unit.

The cultivation body is provided with a chamber-like structure surrounded by a transparent outer wall and having a medium receiving space filled with culture medium on the inside, and has a plurality of air outlets open to the bottom at the top, and a cover having an open-closed structure is provided, and an air supply module is connected to one side of the cultivation body and supplies air sucked in from the outside to the culture medium filled in the medium receiving space.

Additionally, the light irradiation module irradiates light having a predetermined wavelength and intensity to the culture medium filled in the medium receiving space.

The microalgae harvesting unit is installed connected to one side of the cultivation body or is built into the medium receiving space in the cultivation body so that after specific microalgae spawn is inoculated into the culture medium filled in the medium receiving space, the cultured microalgae can be filtered and separated from the culture medium by using air supplied through the air supply module and light irradiated through the light irradiation module to harvest the microalgae.

In the microalgae cultivation device (100), the culture medium is initially transparent, but as carbon is captured and microalgae are cultured, it gradually turns green over time. Such green organisms are microalgae.

The consumer terminal (200) is interconnected with the microalgae cultivation device (100) by establishing a wireless communication network, and calculates carbon reduction information based on the change in carbon dioxide or microalgae harvest amount received from the microalgae cultivation device (100).

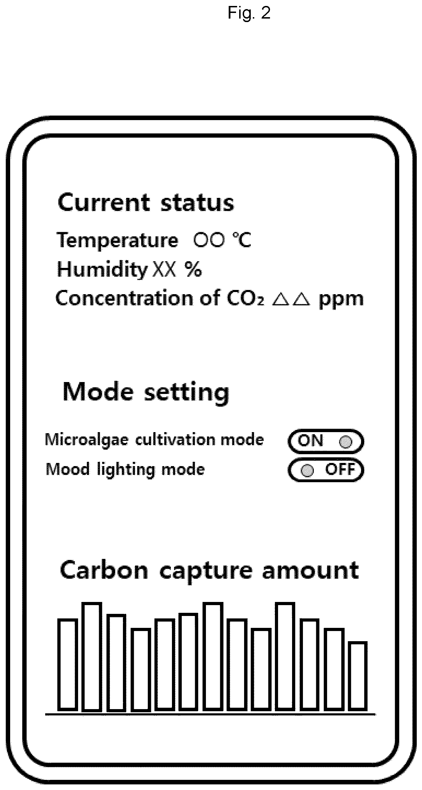

The consumer terminal (200) may be in the form of a smartphone so that the consumer can easily manage it, as shown in FIG. 2, and can monitor the microalgae culture amount and carbon capture data and control the mode by updating the status of the microalgae culture device in real time.

In the case of the mode of the microalgae cultivation device (100), the mood lighting function can be provided through the equipped light irradiation module, so the mode can be selectively selected.

It is also possible to provide a microalgae and environment-related community that can provide various information to consumers through the consumer terminal (200).

The business server (300) provides a reward to the consumer terminal (200) based on the carbon reduction information received through the consumer terminal (200), and stores the carbon reduction information in the blockchain (400).

In an embodiment of the present invention, a business server (300) is connected to a plurality of consumer terminals (200) by establishing a wireless communication network and manages the consumer terminals (200).

The business server (300) can also be directly connected to each microalgae cultivation device (100) by establishing a wireless communication network.

Blockchain (400) is a distributed data storage technology that stores data in blocks, links them in a chain form, replicates them on numerous computers simultaneously, and stores them, making information unique and preventing data forgery or modification by comparing them.

That is, carbon reduction information received by each consumer terminal (200) and reward information provided to the consumer terminal (200) are stored in the blockchain (400) to prevent forgery or falsification.

The reason for preventing forgery or falsification is that the reward provided to the consumer terminal (200) is preferably variable according to the market price, and the reward provided to the consumer terminal (200) can act like cash in the form of points.

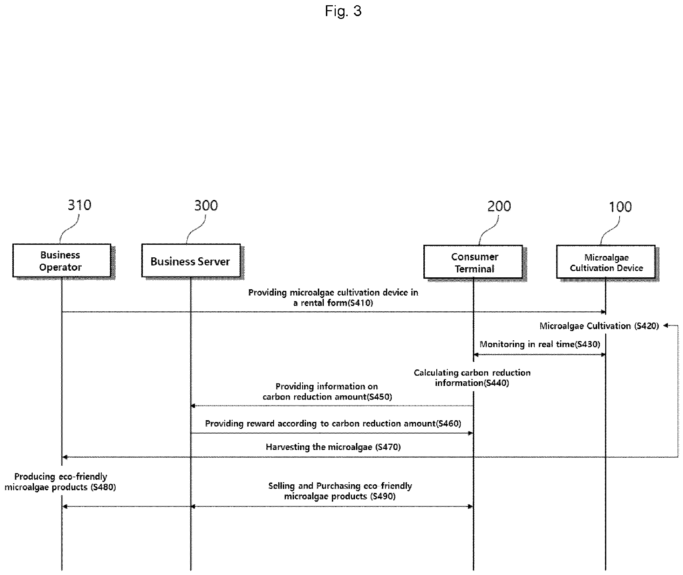

Figure 3 is a flow chart showing a carbon reduction eco-friendly business method utilizing microalgae according to an embodiment of the present invention.

The business provides a microalgae cultivation device to the consumer (S410), and the consumer places the microalgae cultivation device in the living space to capture carbon through the microalgae cultivation device (100) and cultivates microalgae (S420).

At this time, the microalgae cultivation device (100) can be managed by the business server (300) and provided to consumers in a rental form to enable the business to harvest microalgae.

Here, microalgae have a carbon capture capacity of 1:1.8, which is higher than any other plant, and microalgae have a carbon reduction effect about 4 times that of forests.

Next, the consumer terminal (200) monitors the microalgae cultivation device (100) in real time (S430) and calculates carbon reduction information based on the change in carbon dioxide concentration in the living space due to the cultivation of microalgae or the amount of microalgae harvested (S440).

Next, the business server (300) receives information on changes in carbon dioxide concentration (or microalgae cultivation data) and carbon reduction amount from the consumer terminal (200) (S450), stores it in the blockchain, and provides a reward according to the carbon reduction amount to the consumer terminal (200) (S460).

At this time, the business server (300) forms a blockchain smart contract based on the change in carbon dioxide concentration (or microalgae cultivation data) and carbon reduction amount received from the consumer terminal (200), and stores the carbon reduction amount in the blockchain to prevent data falsification and transparently prove carbon reduction data.

Next, the business operator (310) operating the business server (300) harvests the microalgae cultured in the microalgae culture device (100) (S470).

Microalgae harvesting means harvesting microalgae cultured in a microalgae culture device (100) by separating them from the culture medium based on a filtering operation.

After the business operator harvests the microalgae from the microalgae cultivation device (100), the microalgae cultivation (S420) can be started again, and the activities of cultivating the microalgae in the microalgae cultivation device and harvesting the cultured microalgae can be repeated between the consumer and the business operator to form an environmentally friendly virtuous cycle structure.

Next, the business operator (310) produces (S480) an eco-friendly microalgae product using the harvested microalgae.

And, the business operator (310) sells eco-friendly microalgae products online and offline, and consumers with consumer terminals (200) that receive rewards according to carbon reduction can purchase eco-friendly microalgae products with rewards (S490).

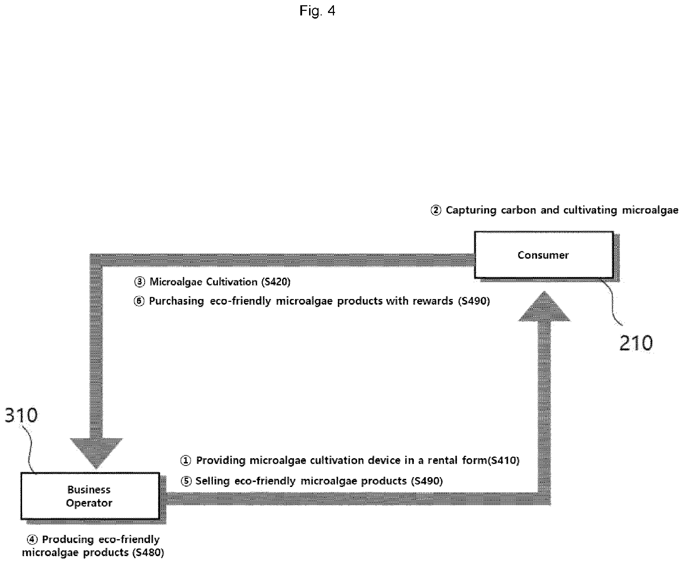

Figure 4 is a diagram showing a circulation structure between consumers and business operators in a carbon reduction eco-friendly business method utilizing microalgae according to an embodiment of the present invention.

Data is transmitted and received between a consumer terminal (200) and a business server (300) through a communication network. However, as shown in Fig. 4, the relationship structure between a business (310) and a consumer (210) that occurs offline as part of an eco-friendly business for carbon reduction using microalgae between the consumer (210) and the business (310) is as follows.

By renting ① a microalgae cultivation device to a consumer (210) and placing the microalgae cultivation device in the consumer's living space, the business (310) can be provided with a place for cultivating microalgae.

In addition, consumers (210) can practice carbon neutrality by capturing carbon dioxide ② through a microalgae cultivation device (100) and cultivating microalgae to obtain the effect of air purification. In addition, consumers (210) are provided with rewards according to the amount of carbon reduction.

The business operator (310) harvests ③ microalgae cultured in a microalgae cultivation device. The harvest can be done by moving directly to a location where a microalgae cultivation device is installed.

By harvesting microalgae from consumers (210), the business operator (310) obtains raw materials for producing eco-friendly microalgae products.

Thereafter, the business operator (310) who harvested the microalgae produces various ④ eco-friendly microalgae products using the harvested microalgae, and the business operator (310) can sell the produced eco-friendly microalgae products to consumers (210) through at least one of online and offline methods to obtain sales revenue.

Microalgae products can be produced in a variety of ways, including cosmetics, beauty products, feed, and health functional foods.

At this time, when purchasing an eco-friendly microalgae product, the consumer (210) can purchase the product as a reward according to the amount of carbon reduction provided by microalgae cultivation.

Since eco-friendly microalgae products can be purchased with rewards in the form of points, consumers (210) can purchase eco-friendly products economically.

In this way, an eco-friendly virtuous cycle business structure can be established in which business operators (310) and consumers (210) can each take what they need from each other and also provide environmental assistance.

Therefore, the present invention has the effect of providing rewards to consumers through air purification and carbon reduction in the living space where consumers live through microalgae cultivation.

In addition, the present invention harvests cultured microalgae to produce eco-friendly products using microalgae, and consumers can purchase eco-friendly products using rewards, thereby creating a virtuous cycle.

In addition, the present invention has the effect of preventing falsification of data by storing microalgae culture data and reduced carbon amount in a blockchain, thereby transparently proving carbon reduction data.

## Claims

1. A carbon reduction eco-friendly business system utilizing microalgae, comprising:
a microalgae cultivation device equipped with a plurality of consumers to capture carbon and cultivate microalgae;
a consumer terminal interconnected by establishing a wireless communication network with the microalgae cultivation device and calculating carbon reduction information based on at least one of a change in carbon concentration, a change in carbon dioxide concentration, and a microalgae harvest amount received from the microalgae cultivation device; and
a business server providing a reward to the consumer terminal based on the carbon reduction information received through the consumer terminal and stores the carbon reduction information in a blockchain;
Wherein, when the business operator operating the business server harvests microalgae from the microalgae cultivation devices of the plurality of consumers and produces an eco-friendly microalgae product using the same, the plurality of consumers purchase the microalgae product using the reward provided to the consumer terminal, thereby establishing a virtuous cycle business structure.

2. The carbon reduction eco-friendly business system utilizing microalgae of claim 1,
wherein the change in carbon concentration is detected by detecting a change between the concentration of carbon contained in air supplied to the microalgae culture device and the concentration of carbon contained in air discharged through the microalgae culture device.

3. The carbon reduction eco-friendly business system utilizing microalgae of claim 1,
wherein the change in carbon dioxide concentration is detected by detecting a change in carbon dioxide concentration in a living space where the microalgae cultivation device is installed while the microalgae cultivation device performs microalgae cultivation.

4. The carbon reduction eco-friendly business system utilizing microalgae of claim 1,
where the microalgae cultivation device accommodates a culture medium prepared for culturing microalgae in the form of an aqueous liquid or solid inside, inoculates a specific microalgae spawn into the accommodated culture medium, and performs cultivation and harvesting of the specific microalgae spawn by providing air and light, thereby generating information on at least one of a change in carbon concentration, a change in carbon dioxide concentration, and a microalgae yield.

5. The carbon reduction eco-friendly business system utilizing microalgae of claim 1,
wherein the consumer terminal updates the status of the microalgae cultivation device in real time, monitors the microalgae cultivation amount and carbon capture data, and controls the mode.

6. The carbon reduction eco-friendly business system utilizing microalgae of claim 1,
wherein the consumer terminal monitors the microalgae cultivation device according to cultivation standard information on the change pattern of appropriate cultivation conditions according to the cultivation status and cultivation time of each type of microalgae and the harvest time, and shares information on the microalgae cultivation device according to the cultivation standard information with the business server.

7. A carbon reduction eco-friendly business method utilizing microalgae, comprising the steps of:
capturing carbon through a microalgae cultivation device at a location designated by a consumer and cultivating microalgae;
calculating carbon reduction information based on at least one of a change in carbon concentration, a change in carbon dioxide concentration, and a microalgae harvest amount due to the cultivation of the microalgae;
storing the carbon reduction information in a blockchain by a business server; and providing a reward according to the carbon reduction amount to the consumer terminal;
a step in which the business operator operating the business server harvests the microalgae cultured in the microalgae cultivation device, produces eco-friendly microalgae products using the harvested microalgae, and sells them online and offline; and
a step in which the consumer purchases the microalgae product with the reward provided to the consumer terminal.

8. The carbon reduction eco-friendly business method utilizing microalgae of claim 7,
wherein the reward provided to the consumer terminal is in the form of points.

9. The carbon reduction eco-friendly business method utilizing microalgae of claim 7,
wherein the microalgae cultivation device is managed by a business server and provided in a rental form to enable the business to harvest microalgae.
